# EUROPÄISCHE PATENTSCHRIFT

(11) **EP 1 432 380 B1**
(45) Veröffentlichungstag und Bekanntmachung des Hinweises auf die Patenterteilung: **27.09.2006**
(21) Anmeldenummer: 02794780.3
(22) Anmeldetag: 12.08.2002
(51) Int. Cl.: A61K 6/033, A61K 6/04, A61L 27/06

(54) **VORRICHTUNG AUF BASIS VON NITINOL MIT POLYPHOSPHAZENÜBERZUG**
DEVICE BASED ON NITINOL WITH A POLYPHOSPHAZENE COATING
DISPOSITIF A BASE DE NITINOL COMPORTANT UN REVETEMENT POLYPHOSPHATE

(30) Priorität: 17.08.2001 DE 10140522; 23.01.2002 DE 10202467
(43) Veröffentlichungstag der Anmeldung: 30.06.2004
(73) Patentinhaber: Polyzenix GmbH, 89077 Ulm (DE); CeloNova BioSciences, Newnan, GA 36403 (US)
(72) Erfinder: SCHÜSSLER, Andreas, 76327 Pfinztal (DE); GRUNZE, Michael, 69151 Neckargemünd (DE); DENK, Roman, 89197 Weidenstetten (DE)
(74) Vertreter: Teipel, Stephan
(86) Internationale Anmeldenummer: PCT/EP2002/009017
(87) Internationale Veröffentlichungsnummer: WO 2003/015719

(56) Entgegenhaltungen:
- WO-A-01/87368
- WO-A-02/24247
- WO-A-99/09088
- WO-A-99/42147
- DE-A- 19 613 048
- US-A- 5 634 946
- US-A- 5 873 904
- US-A1- 2001 014 717
- US-B1- 6 254 634

## Beschreibung

Die vorliegende Erfindung betrifft einen vaskulären Stent, umfassend ein Substrat auf Basis von Nitinol und darauf angeordnet mindestens teilweise einen Überzug bzw. eine Beschichtung auf der Basis von mindestens einem Polyphosphazenderivat mit der allgemeinen Formel (I) und ein Verfahren zu dessen Herstellung.

Der Werkstoff Nitinol ist eine intermetallische Verbindung, hauptsächlich bestehend aus den Metallen Nickel und Titan. Nitinol weist die Besonderheit auf, nach vorangegangener Verformung bei einer niedrigen Temperatur seine ursprüngliche Form nach Erwärmung zu einer höheren Temperatur wieder anzunehmen. Diese Eigenschaft ist auch als "thermisches Formgedächtnis" oder "Thermal Shape Memory" bekannt. Darüber hinaus weist Nitinol eine zweite funktionelle Eigenschaft auf, das "mechanische Formgedächtnis" oder auch Superelastizität. Insbesondere die letztere Eigenschaft, das Vermögen elastische Dehnungen bis 8% völlig reversibel zu ertragen, hat zu einer Vielzahl von Anwendungen als Implantate und Komponenten für interventionelle Behandlungsmethoden, wie zum Beispiel Stents, Stent Grafts, vaskulare Verbinder, Filter, Fangkörbchen und Führungsdrähte geführt. Stents und Filter werden üblicherweise mit einem Einführbesteck an die Stelle des Gefäßes gebracht, an welcher sie sich zu der ursprünglichen Form entfalten. Zum Einsetzen in das Einführsystem wird das Nitinol-Bauteil in der Kälte bei Dehnungen bis zu etwa 8% komprimiert. So werden beispielsweise endovaskuläre Stents mit einem definierten Durchmesser zunächst bearbeitet, dann in der Kälte komprimiert und mittels eines Einführbestecks an die Stelle der Ader gebracht, an der sie platziert werden sollen. An der gewünschten Stelle entfaltet sich der vorher komprimierte Stent durch die Körperwärme und/oder durch sein elastisches Vermögen zu seiner ursprünglichen Form und stützt die umliegende Ader. Durch die bereits bei der Fertigung festgelegte Form kann ein Überdehnen der Ader vermieden werden.

Der Werkstoff Nitinol weist an sich eine sehr gute Körperverträglichkeit auf. Um ein möglicherweise auftretendes "Ausbluten" von Nickel bzw. Nickelionen von vorneherein zu vermeiden und um eine besonders gleichmäßige Oberflächenstruktur zu erzielen, wurden verschiedene Techniken entwickelt, um die Oberfläche eines im wesentlichen aus Nitinol gefertigten Implantats, wie beispielsweise einen Stent, zu versiegeln und mit einer dichten, nickelundurchlässigen Schicht zu versehen. Diese Verfahren der chemischen Nachbehandlung führen üblicherweise zu einer dichten Schicht aus Titandioxid, durch welche Nickel kaum austreten kann. Diese Verfahren sind unter anderem als Elektropolitur bekannt und in unterschiedlichen Varianten üblich.

Oxidische Schichten verschlechtern zwar nicht zwangsläufig die Körperverträglichkeit von Implantaten, jedoch liefern sie eine Oberfläche, welche nur eine geringe Hämokompatibilität erwarten lässt und so bei der Behandlung von Patienten, die ein solches Implantat erhalten, den Einsatz von Vitamin K Antagonisten sowie anderen gerinnungshemmenden Mitteln, wie Acetylsalicylsäure, notwendig macht. Dieses stellt eine zusätzliche Belastung für den betroffenen Patienten dar.

Zudem kann das Verfahren des elektrochemischen Polierens nicht überall eingesetzt werden. An Bereichen bzw. Stellen, an welchen das elektrochemische Polieren nicht angewendet werden kann, wie beispielweise bei Implantaten, die aus einer Kombination verschiedener Werkstoffe bestehen und sich demzufolge beim elektrochemischen Polieren diese selektiv auflösen würden, bieten natürliche Oxidschichten einen schlechteren Schutz gegen den Austrag von Metallionen und demzufolge eine geringere Blut- und Körperverträglichkeit.

Wünschenswert wäre also eine zusätzlich anzuwendende Form der Oberflächenveredelung wie beispielsweise eine Beschichtung, welche die Eigenschaften einer durch Elektropolitur oder anderweitig erzeugten dichten und für Metallionen, insbesondere Nickelionen, undurchlässigen Oberfläche im Hinblick auf die Hämo- und Biokompatibilität und auch andere Eigenschaften, wie insbesondere die Gleitfähigkeit, weiter verbessert, ohne die Dichtigkeit und isolierenden Eigenschaften einer solchen Oberfläche zu schädigen.

Aus dem Stand der Technik sind eine Fülle von Materialien bekannt und untersucht, die für die Herstellung von solchen Beschichtungen Verwendung finden. So ist beispielsweise aus der WO 98/56312 eine expandierbare Hülle aus ε-PTFE bekannt, die auch für die Züchtung von künstlichen Adern verwendet werden kann. Andere Materialien für diese Verwendung sind in der EP-A-0-810 845 angeführt, worin beispielsweise Polymere genannt werden, die in US-A-4,883,699 und US-A-4,911,691 beschrieben werden. Weitere Polymere für den genannten Zweck sind z.B. hydrolysiertes Polyacrylnitril (US-A-4,480,642), hydrophile Polyether (US-A-4,798,876) und Polyurethanediacrylate (US-A-4,424,395). Weiterhin sind verschiedene Hydrogele bekannt, die als Beschichtung für diesen Zweck eingesetzt werden können. Die Reihe der potentiell anwendbaren Materialien kann weiter durch Polyvinylpyrrolidon(PVP), Polyvinylalkohole (PVA), Polyethylen-oxid (PEO) und Polyhydroxyethylmethacrylate p(HEMA) ergänzt werden. Ferner ist im Stand der Technik die Anwendung einer Reihe von Standardmaterialien wie Polyurethane, Polyethylene und Polypropylene als mögliche Werkstoffe beschrieben. Ebenso sind Mischungen dieser Werkstoffe untereinander zur Beschichtung von Implanaten bekannt. Eine Reihe weiterer Materialien ist zudem aus EP-A-0-804 909 bekannt.

Die Eigenschaften dieser Werkstoffe sind unterschiedlich und es kann davon ausgegangen werden, dass jeder dieser Werkstoffe besondere Eigenschaften für bestimmte Anwendungen in der Beschichtung von künstlichen Implantaten und anderen medizinischen Vorrichtungen aufweist. So ist beispielsweise PVA sehr gut in Flüssigkeiten löslich und wird schnell resorbiert. Andere Materialien weisen eine gute Blutverträglichkeit auf. Wiederum andere Materialien sind besonders gut streckbar. Jedoch weisen alle Materialien Nachteile in bestimmten Bereichen auf. PVA zeigt zum Beispiel keine gute Blutverträglichkeit. ε-PTFE ist beispielsweise sehr gut streckbar und weist auch eine gute Blutverträglichkeit auf, jedoch ist dieses Material ausgesprochen schwierig zu handhaben und die Herstellung solcher Beschichtungen erfordert eine Reihe von Verarbeitungsschritten (vgl. WO 96/00103). Auch ist eine derart beschaffene Oberfläche nur in Grenzen temperaturstabil und wird in der Kälte spröde, was im speziellen Fall der Beschichtung von Nitinol zumindest zu Haarrissen führt, an denen sich Thromben bilden können. Zudem ist die Haftung einer solchen Beschichtung nicht besonders gut. Diese wird bei der notwendigen Kältebehandlung spröde und löst sich dadurch sehr leicht ab, was insbesondere bei Implantaten in endovaskulären Systemen erhebliche gesundheitliche Folgen haben kann. Bei anderen Materialien können elastische Eigenschaften, die für eine Beschichtung der genannten Art in praktisch allen Fällen wichtig sind, nur durch die Zugabe von Weichmachern erreicht werden. Weichmacher vermindern jedoch die Blut- und Körperverträglichkeit erheblich. Ferner kann ein Ausschwemmen der Weichmacher zusätzlich zu Entzündungsreaktion, Reaktionen des Immun- und Gerinnungssystems und damit zu einer Belastung für den Patienten führen. Grundsätzlich wird jedoch die Mehrzahl derart "weich" gemachter Kunststoffe und Kunststoffgemische in der Kälte spröde und verliert seine Elastizität. Dadurch ist bei solchen Materialien immer die Gefahr vorhanden, dass sich beim erneuten Expandieren Risse bilden oder sich die Schicht ablöst.

Weitere bekannte Komplikationen, die bei einer solchen Beschichtung auftreten können, stehen in Zusammenhang mit den aus dem Abbau einiger für die Beschichtung verwendeter Stoffe und den hier entstehenden Abbauprodukten. So ist beispielsweise bekannt, dass bei der Auflösung respektive der Resorption und der Zersetzung einiger der aus dem Stand der Technik bekannten Beschichtungsstoffe Entzündungsreaktionen auftreten können (van der Giessen, Circulation Vol. 94, Nr. 7. 1996). Dieses Problem ergibt sich entweder durch die teilweise mangelhafte Verträglichkeit solcher Beschichtungen oder aber durch Reaktion auf Zersetzungsprodukte, die bei der Zersetzung der genannten Stoffe entstehen. Dieses liegt insbesondere daran, dass sich die Stoffe teilweise nur wenig kontrolliert aufbringen lassen und somit an unterschiedlichen Stellen unterschiedlich stark zersetzt oder abgebaut werden.

Ferner spielt für die problemfreie Handhabung derartiger beschichteter Implantate das Verhalten gegenüber Bakterien, Makrophagen und Proteinen, die sich auf den Oberflächen der Implantate und Vorrichtungen ablagern, eine zentrale Rolle, da insbesondere diese Ablagerungen zu Entzündungen und anderen Problemen beim Einwachsen der Implantate führen können. Insbesondere bei der Herstellung von Gefäßimplantaten, wie vaskuläre und nicht vaskuläre Stents, Hernia Patches, Urether, Filter, vaskuläre Verbinder sowie Implantate im Mund-, Zahn- und Rachenbereich, sind die genannten Eigenschaften und Besonderheiten der Werkstoffe ein wesentlicher Aspekt. Dieses ist darin begründet, dass durch Temperatureffekte bei der Verarbeitung von Nitinol hervorgerufene Risse Ansatzpunkte für eine verstärkte Thrombenbildung und andere Ablagerungen wie Proteine, Makrophagen, etc., darstellen, welche nach Implantation zu einem Risiko für den Patienten werden können.

DE 196 13 048 A1 beschreibt künstliche Implantate mit antithrombogenen Eigenschaften, umfassend ein Implantatmaterial als Substrat und einen mindestens teilweise auf die Oberfläche des Substrats aufgebrachten biokompatiblen Überzug, der ein antithrombogenes Polymer auf Polyphosphazen-Basis enthält. US-A-5,873,904 beschreibt eine medizinische Vorrichtung zur Einführung in vaskuläre Systeme, worin mindestens eine Schicht auf Basis eines bioaktiven Materials auf einer Oberflächenstruktur angeordnet ist und über der Schicht aus bioaktivem Material eine poröse Schicht vorgesehen ist, die vorzugsweise auf Basis von Polyamid, Parylen und/oder einem Parylenderivat ist. WO 01/87368 A1 beschreibt ein Verfahren zur Beschichtung von Stents und anderen medizinischen Vorrichtungen mit einer dünnen Polymerfolie, wobei als filmbildende Polymere aliphatische Polyester, Poly(aminosäuren), Copoly(ether-ester), Polyalkylenoxalate, Polyamide, Poly(iminocarbonate), Polyorthoester, Polyoxaester, Polyamidoester, Amidogruppen enthaltende Polyoxaester, Poly(anhydride), Polyphosphazene, Biomoleküle und Blends davon genannt werden. Ferner werden als zu beschichtende Substrate biokompatible Metalle einschließlich rostfreiem Stahl, Tantal, Titanlegierungen wie Nitinol und Cobaltlegierungen wie Cobalt-Chrom-Nickel-Legierungen, nicht-metallische biokompatible Materialien, einschließlich Polyamide, Polyolefine, Polyester und aliphatische Polyester, genannt. WO 99/42147 A1 beschreibt eine bioabbaubare Polymerzusammensetzung, die sich durch ein "thermisches Formgedächtnis" auszeichnet.

Somit liegt der vorliegenden Erfindung die Aufgabe zugrunde, eine als künstliches Implantat geeignete Vorrichtung auf Basis von Nitinol bereitzustellen, die sich durch eine hohe Körperverträglichkeit und eine sehr gute Hämokompatibilität auszeichnen soll. Zusätzlich sollte eine derartige Vorrichtung die erheblichen Temperaturschwankungen sowie mechanischen Belastungen, wie beispielsweise elastische Dehnungen von bis zu etwa 8%, die bei der Verarbeitung und Anwendung des Werkstoffes Nitinol auftreten, ohne eine Verschlechterung des Eigenschaftsprofils der Vorrichtung, wie z.B. Ablösung oder Rißbildung, überstehen. Dieses sollte insbesondere ohne die Anwendung eines Weichmachers erreicht werden, da ansonsten die Körperverträglichkeit eines solchen Implantats erheblich vermindert werden würde.

Diese Aufgabe wird durch die in den Ansprüchen gekennzeichneten Ausführungsformen gelöst. Insbesondere wird ein vaskulären Stent bereitgestellt, umfassend ein Substrat auf der Basis von Nitinol und darauf angeordnet mindestens teilweise einen Überzug bzw. eine Beschichtung auf der Basis von mindestens einem Polymer mit der folgenden allgemeinen Formel (I), 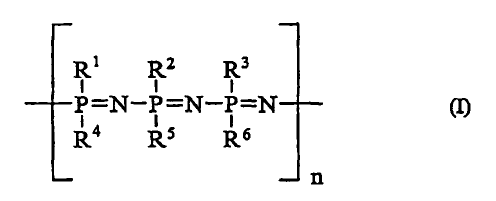 worin n für 2 bis ∞ steht, die Reste R¹ bis R⁶ gleich oder unterschiedlich sind und einen Alkoxy-, Alkylsulfonyl-, Dialkylamino- oder Aryloxyrest oder einen Heterocycloalkyl- oder Heteroarylrest mit Stickstoff als Heteroatom bedeuten.

Überraschenderweise weist der erfindungsgemäße Stent eine hohe Körperverträglichkeit und eine sehr gute Hämokompatibilität auf. Zudem zeigt der erfindungsgemäße Stent im wesentlichen keine Adsorption von Makrophagen bzw. Ansiedelung von Bakterien. Das im Rahmen der vorliegenden Erfindung zur Beschichtung des Substrats auf Nitinol-Basis spezifisch ausgewählte Polyphosphazen mit der vorstehenden Formel (I) hält in besonders vorteilhafter Weise die teilweise extremen Temparaturschwankungen und mechanischen Belastungen im Verlauf der Bearbeitung des Nitinolsubstrats aus, ohne spröde zu werden, sich abzulösen oder zu reißen. Durch das spezifisch ausgewählte Polyphosphazen mit der vorstehenden Formel (I) werden die Obefiächeneigenschaften von Nitinol dauerhaft verbessert, da das spezifisch ausgewählte Polyphosphazenderivat gemäß der allgemeinen Formel (I) weder hydrolytisch noch enzymatisch abgebaut wird. In vorteilhafter Weise ist das spezifisch ausgewählte Polyphosphazen mit der vorstehenden Formel (I) über einen sehr weiten Temperaturbereich, insbesondere bei Temperaturen unter dem Gefrierpunkt, ohne den Einsatz von Weichmachern elastisch.

Der Polymerisationsgrad des für die Beschichtung des Nitinolsubstrats verwendeten Polymers gemäß der vorstehenden Formel (I) beträgt 2 bis ∞. Bevorzugt ist ein Bereich für den Polymerisationsgrad von 20 bis 200.000, mehr bevorzugt von 40 bis 100.000.

Vorzugsweise ist mindestens einer der Reste R¹ bis R⁶ im verwendeten Polymer ein Alkoxyrest, der mit mindestens einem Fluoratom substituiert ist.

Die Alkylreste in den Alkoxy-, Alkylsulfonyl- und Dialkylaminoresten sind beispielsweise gerad- oder verzweigtkettige Alkylreste mit 1 bis 20 Kohlenstoffatomen, wobei die Alkylreste beispielsweise mit mindestens einem Halogenatom, wie ein Fluoratom, substituiert sein können.

Beispiele für Alkoxyreste sind Methoxy-, Ethoxy-, Propoxy- und Butoxygruppen, die vorzugsweise mit mindestens einem Fluoratom substituiert sein können. Besonders bevorzugt ist die 2,2,2-Trifluoroethoxygruppe. Beispiele für Alkylsulfonylreste sind Methyl-, Ethyl-, Propyl- und Butylsulfonylgruppen. Beispiele für Dialkylaminoreste sind Dimethyl-, Diethyl-, Dipropyl- und Dibutylaminogruppen.

Der Arylrest im Aryloxyrest ist beispielsweise eine Aryleinheit mit einem oder mehreren aromatischen Ringsystemen, wobei der Arylrest beispielsweise mit mindestens einem, vorstehend definierten Alkylrest substituiert sein kann. Beispiele für Aryloxyreste sind Phenoxy- und Naphthoxygruppen und Derivate davon.

Der Heterocycloalkylrest ist beispielsweise ein 3- bis 7-Atome enthaltendes Ringsystem, wobei mindestens ein Ringatom ein Stickstoffatom ist. Der Heterocycloalkylrest kann beispielsweise mit mindestens einem, vorstehend definierten Alkylrest substituiert sein. Beispiele für Heterocycloalkylreste sind Piperidinyl-, Piperazinyl-, Pyrrolidinyl- und Morpholinylgruppen und Derivate davon. Der Heteroarylrest ist beispielsweise eine Verbindung mit einem oder mehreren aromatischen Ringsystemen, wobei mindestens ein Ringatom ein Stickstoffatom ist. Der Heteroarylrest kann beispielsweise mit mindestens einem, vorstehend definierten Alkylrest substituiert sein. Beispiele für Heteroarylreste sind Pyrrolyl-, Pyridinyl-, Pyridinolyl-, Isochinolinyl- und Chinolinylgruppen, und Derivate davon.

In einer bevorzugten Ausführungsform der vorliegenden Erfindung ist das Polymer Poly[bis(trifluoroethoxy)phosphazen]. Das Polymer kann auch ein mit ³²P-, ³³P- oder As- oder Sb-Isotopen markiertes Poly[bis(trifluoro-ethoxy)phosphazen] sein.

Die polymere Verbindung Poly[bis(trifluoroethoxy)phosphazen] zeigt als Volumenmaterial eine gute antithrombogene Wirkung (vgl. Tur, Untersuchungen zur Thrombenresistenz von Poly[bis(trifluoroethoxy)phosphazen) und Hollemann Wiberg, "Stickstoffverbindungen des Phosphors", Lehrbuch der anorganischen Chemie, 666-669, 91.-100. Auflage, Walter de Gruyter Verlag, 1985, sowie Tur, Vinogradova, u.a. "Entwicklungstendenzen bei polymeranalogen Umsetzungen von Polyphosphazen", Acta Polymerica 39, 424-429, Nr. 8, 1988. In DE 196 13 048 A1 wurde die fluorierte Form von Polyphosphazenen als Beschichtung für die Beschichtung künstlicher Implantate eingesetzt, wobei ausdrücklich Herzklappen genannt werden. Solche Implantate werden jedoch nicht aus Nitinol hergestellt Zudem findet sich in DE 196 13 048 A1 keine Offenbarung bezüglich des Aspekts der extremen Temperaturbelastungen bei Nitinolimplantaten.

Die erfindungsgemä&en Stents weisen zum einen ausgezeichnete mechanische und körperverträgliche Eigenschaften und zum anderen eine hohe Resistenz gegenüber entzündungsfördemden Organismen, wie z.B. Makrophagen, auf. Durch die Beschichtung der Nitinoloberfläche mit dem spezifisch ausgewählten Polyphosphazen mit der vorstehenden Formel (I) wird einerseits die Hämo- und Biokompatibilität der genannten Oberfläche verbessert, andererseits wird bei der Aufbringung der Beschichtung bzw. des Überzugs die durch Elektropolitur oder anderweitige Techniken generierte, die Nitinoloberfläche versiegelnde Oberflächenschicht nicht beschädigt. Somit können mögliche Folgeschäden nach der Implantation reduziert werden.

Im Rahmen der vorliegenden Erfindung kann das Substrat auf Basis von Nitinol teilweise mit einem Überzug auf Basis von mindestens einem Polymer mit der allgemeinen Formel (I) beschichtet sein. Üblicherweise ist es jedoch bevorzugt, daß das Substrat auf Basis von Nitinol vollständig mit einem Überzug auf Basis von mindestens einem Polymer mit der allgemeinen Formel (I) beschichtet ist.

In einer Ausführungsform der vorliegenden Erfindung liegt das Nitinolsubstrat, welches darauf beschichtet einen Überzug aus dem Polyphosphazenpolymer gemäß der Formel (I) aufweist, in der Form einer zumindest teilweise durchbrochenen bzw. perforierten Röhre vor, wie es beispielsweise bei einem endovaskulären Implantat, wie einem Stent, üblich ist.

Der gemäß der vorliegenden Erfindung auf dem Nitinolsubstrat angeordnete bzw. abgeschiedene Überzug aus mindestens einem Polyphosphazenpolymer gemäß der Formel (I) kann femer ein oder mehrere pharmazeutisch wirksame Verbindungen, wie beispielsweise zelltoxische Verbindungen, wie Rapamycin, Taxol, Doxorubicin, Platinderivate, 5-Fluoracyl oder entzündungshemmende Wirkstoffe wie Diclofenac oder Acetylsalicylsäure oder andere Wirkstoffe in entsprechender galenischer Zubereitung, vorzugsweise mit verzögerter Freisetzung bzw. quantitativ kontrollierter Freisetzung über einen längeren Zeitraum, umfassen.

Der Überzug des erfindungsgemäßen Stents weist beispielsweise eine Dicke von etwa 1 nm bis etwa 100 µm, vorzugsweise bis etwa 500 nm und besonders bevorzugt bis etwa 100 nm auf.

In einer Ausführungsform des erfindungsgemäßen Stents ist zwischen der Oberfläche des Substrats und dem aus dem Polyphosphazenderivat aufgebauten Überzug eine Schicht angeordnet, die einen Adhäsionspromotor enthält, d.h. an den Überzug auf Basis mindestens eines Polymers mit der allgemeinen Formel (I) angrenzend ist eine Adhäsionspromotorschicht angeordnet.

Der Adhäsionspromotor bzw. Spacer enthält bevorzugt eine polare Endgruppe. Beispiele hierfür sind Hydroxy-, Carboxylat-, Aldehyd-, Amino- oder Nitrogruppen. Es könne aber auch Endgruppen auf Basis eines Alkoxy-, Alkylsulfonyl-, Dialkylamino- oder Aryloxyrestes oder eines Heterocycloalkyl- oder Heteroarylrestes mit Stickstoff als Heteroatom vorliegen, wobei diese Reste auch unterschiedlich, beispielsweise durch Halogenatome, insbesondere Fluor substituiert sein können.

Insbesondere kann der Adhäsionspromotor beispielsweise eine Siliciumorganische Verbindung, vorzugsweise ein aminoterminiertes Silan bzw. eine Verbindung auf Basis eines Aminosilans, ein aminoterminiertes Alken, ein nitroterminiertes Alken, ein nitroterminiertes Silan oder eine Alkylphosphonsäure sein. Als Adhäsionspromotor sind Aminopropyltrimethoxysilan und Aminopropyltriethoxysilan besonders bevorzugt.

Der Adhäsionspromotor verbessert insbesondere die Haftung des Überzugs auf der Oberfläche des Nitinolsubstrats durch Kopplung des Adhäsionspromotors an die Nitinoloberfläche, beispielsweise über ionische und/oder kovalente Bindungen, und weitere Kopplung an das beschriebene Polymer der Formel (I) des Überzugs, beispielsweise über ionische und/oder kovalente Bindungen.

Ein weiterer Gegenstand der vorliegenden Erfindung betrifft die Herstellung des erfindungsgemäßen Stents, umfassend die Schritte:
(a) Bereitstellen eines Substrats auf Nitinol-Basis,
(b) Aussetzen der Nitinoloberfläche einer Plasmabehandlung,
(c) gegebenenfalls Hydroxylieren der in Schritt (b) behandelten Oberfläche und Aufbringen eines Adhäsionspromotors und
(d) Beschichten des Substrats mit mindestens einem Polymer der vorstehend angeführten Formel (I).

Das Substrat auf Nitinol-Basis kann in Schritt (a) beispielsweise in Form einer mindestens teilweise durchbrochenen bzw. perforierten Röhre bereitgestellt werden. Das Substrat umfasst im wesentlichen Nitinol als den das Substrat aufbauenden Werkstoff. Das Substrat auf Nitinol-Basis kann dabei bereits einer Elektropolitur oder anderweitigen Behandlung zur Erzeugung einer dichten und für Nickelionen undurchlässigen Oberfläche unterworfen worden sein.

In Schritt (b) des erfindungsgemäßen Verfahrens erfolgt eine Plasmabehandlung der Nitinoloberfläche, wobei das Plasma vorzugsweise ein Luft- oder Sauerstoffplasma ist. Durch die Plasmabehandlung wird auf dem Substrat auf der Basis von im wesentlichen Nitinol eine ültradünne, dichte TiO₂-Schicht erzeugt. Es können auch andere Plasmagase wie beispielsweise Argon eingesetzt werden. Ferner kann das eingesetzte Plasmagas gegebenenfalls ein oder mehrere, im Rahmen eines solchen Verfahrenschritts übliche Zusätze, wie z.B. Allyl, Vinylbenzol, etc., enthalten. In Schritt (b) des erfindungsgemäßen Verfahrens erfolgt mittels der Plasmabehandlung eine Aktivierung und Reinigung der Nitinoloberfläche, wodurch eine vollständige und gleichmäßige Oxidation und Aktivierung der Oberfläche herbeigeführt wird. Gleichzeitig werden vorhandene Verunreinigungen oder Fremdkörper oxidiert und die Oberfläche gereinigt. In diesem Zusammenhang ist zu erwähnen, dass die Aktivierung und Beschichtung der Oberfläche, wie in DE 196 13 048 beschrieben, eine durch Elektropolitur erzeugte Oberfläche abträgt und damit den Austritt von Nickel oder Nickelionen erlaubt. Daher ist die in DE 196 13 048 beschriebenen Vorgehensweise für die Beschichtung von Nitinolimplantaten oder Vorrichtungen ungeeignet und führt zu Implantaten, deren Einsatz eine potentielle Schädigung des Patienten durch die Elution von Nickel bzw. Nickelionen herbeiführt. Weiterhin kommt es durch den in DE 196 13 048 beschriebenen Einsatz von Schwefelsäure zur Bildung von Verbindungen des Typs TiO(SO₄), welche die durch die Oberflächenbehandlung generierte TiO₂ Oberfläche auflöst und damit schädigt und zerstört.

Anschließend wird in Schritt (c) gegebenenfalls eine Hydroxylierung der Nitinoloberfläche, beispielsweise durch Wasser, durchgeführt und ein Adhäsionspromotor, vorzugsweise Aminopropyltrimethoxysilan (APTMS) oder Aminopropyltriethoxysilan, auf die Oberfläche aufgebracht und vemetzt.

Auf diese Oberfläche wird anschließend in Schritt (d) üblicherweise durch Sprühen oder Tauchen eine Lösung, die mindestens eine Verbindung der allgemeinen Formel (I) in einer Konzentration von 0,1 bis 99 Gew.-% enthält, aufgebracht. Als mögliche Lösungsmittel können hier Ethylacetat, Aceton, THF, Toluol oder Xylole verwendet werden. Jedoch ist die Wahl der Lösungsmittel oder Lösungsmittelgemische nicht auf die genannten Lösungsmittel beschränkt.

Das Verdampfen des Lösungsmittels kann ohne weitere Maßnahmen ablaufen. Üblicherweise wird die Konzentration des Lösungsmitteldampfes über dem Substrat, der Druck und die Temperatur kontrolliert. Zu Beginn der ersten Trocknungs-phase wird üblicherweise eine über dem beschichteten Substrat mit Lösungsmitteldampf gesättigte Atmosphäre eingestellt, wobei die Konzentration des Lösungsmitteldampfes anschließend über mehrere Stunden langsam reduziert wird. Die Temperatur kann dabei von -30°C bis + 90°C variieren. Der Druck kann während der ersten Trocknungsphase eine Rampe von Normaldruck bis Wasserstrahlvakuum (20 Torr) durchlaufen. Nach der ersten Trocknungsphase wird das beschichtete Substrat dann für eine bestimmte Zeit im Ölpumpenvakuum (0,1 Torr) weiter getrocknet.

Der mit dem getrockneten Polymer der Verbindung (I) beschichtete Stent auf Basis von Nitinol kann dann nach entsprechender Sterilisation direkt weiter verwendet und in der Kälte weiter bearbeitet, d.h. komprimiert werden. Je nach Konzentration der Lösung der Polymerverbindung (I) und den Bedingungen während der ersten Trocknungsphase können verschiedene Schichtdicken von 0,1 nm bis 300 µm oder dicker, vorzugsweise im Bereich von 500 nm bis 30 µm, und besonders bevorzugt um 100 nm, erzeugt werden.

Die Strukturierung der Polymerbeschichtung unterliegt keiner spezifischen Festlegung. So können Strukturen in der Größenordnung von Nanometem, Mikrometern oder noch größer oder kleiner, vorzugsweise im Bereich von 10 nm bis 100 µm, hergestellt werden. Weiterhin können alle Strukturen hergestellt und verwendet werden, die photolithographisch oder mit Elektronenstrahlen, Röntgenstrahlen oder Laserstrahlen oder mittels anderer Techniken erzeugt werden können.

Eine Mikrostrukturierung der Beschichtung kann auch durch ein direktes "Schreiben" auf einer zuvor auf der Basis von mindestens einem Polyphosphazenderivat gemäß vorstehender Formel (I) hergestellten Folie mittels Laser-, Elektronen- oder Röntgenstrahlen oder aber durch "Schmelzstrukturieren" erhalten werden, wobei ein dünner Draht auf die Schmelztemperatur des Polymers gebracht wird, der dann die gewünschte Struktur durch direkten Kontakt in die Beschichtung einschmilzt. Besondere Vorteile können durch eine derartige Strukturierung dadurch erreicht werden, daß Strukturen in die Folie eingeprägt werden, die das Strömungsverhalten von Flüssigkeiten besonders günstig gestalten (z.B. Haifischhaut oder Lotoseffekt).

## Patentansprüche

1. Vaskulärer Stent, umfassend ein Substrat auf der Basis von Nitinol und darauf angeordnet mindestens teilweise einen Überzug auf Basis von mindestens einem Polymer mit der folgenden allgemeinen Formel (I), 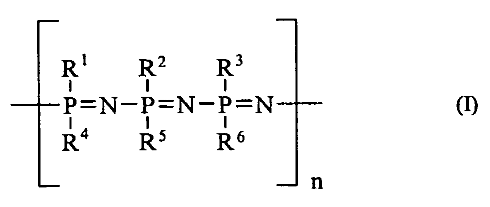 worin n für 2 bis ∞ steht, die Reste R¹ bis R⁶ gleich oder unterschiedlich sind und einen Alkoxy-, Alkylsulfonyl-, Dialkylamino- oder Aryloxyrest oder einen Heterocycloalkyl- oder Heteroarylrest mit Stickstoff als Heteroatom bedeuten.

2. Vaskulärer Stent nach Anspruch 1, wobei auf dem Oberfläche des Substrats auf Nitinol-Basis eine ültradünne TiO₂-Schicht angeordnet ist.

3. Vaskulärer Stent nach Anspruch 1 oder 2, wobei mindestens einer der Reste R¹ bis R⁶ ein Alkoxyrest ist, der mit mindestens einem Fluoratom substituiert ist.

4. Vaskulärer Stent nach einem der Ansprüche 1 bis 3, wobei das Polymer Poly[bis(trifluorethoxy)phosphazen] ist.

5. Vaskulärer Stent nach einem der Ansprüche 1 bis 4, wobei zwischen der Oberfläche des Substrats auf Nitinol-Basis und dem Überzug eine Schicht angeordnet ist, die einen Adhäsionspromotor enthält.

6. Vaskulärer Stent nach Anspruch 5, wobei der Adhäsionspromotor eine polare Endgruppe enthaltende Verbindung, insbesondere eine Silizium-organische Verbindung, ist.

7. Vaskulärer Stent nach Anspruch 6, wobei die Silizium-organische Verbindung Aminopropyltrimethoxysilan ist.

8. Vaskulärer Stent nach einem der Ansprüche 1 bis 7, wobei das Substrat auf Nitinol-Basis vollständig mit einem Überzug auf Basis von mindestens einem Polymer mit der allgemeinen Formel (I) beschichtet ist.

9. Vaskulärer Stent nach einem der vorhergehenden Ansprüche, wobei das Substrat auf Nitinol-Basis in der Form einer zumindest teilweise perforierten Röhre vorliegt.

10. Vaskulärer Stent nach einem der vorhergehenden Ansprüche, wobei der Überzug auf Basis von mindestens einem Polymer mit der allgemeinen Formel (I) mikrostrukturiert ist.

11. Verfahren zur Herstellung eines vaskulären Stents nach einem der Ansprüche 1 bis 10, umfassend die Schritte:
(a) Bereitstellen eines Substrats auf Nitinol-Basis,
(b) Aussetzen des Substrats auf Nitinol-Basis einer Plasmabehandlung,
(c) gegebenenfalls Hydroxylieren der in Schritt (b) behandelten Oberfläche und Aufbringen eines Adhäsionspromotors und
(d) Beschichten des Substrats mit mindestens einem Polymer gemäß Formel (I), wie in einem der vorhergehenden Ansprüche definiert.

12. Verfahren nach Anspruch 11, wobei das Plasma in Schritt (b) ein Luft- oder Sauerstoffplasma ist.

13. Verfahren nach Anspruch 11 oder 12, wobei nach Schritt (d) die Polymerbeschichtung mittels Laser-, Elektronen- oder Röntgenstrahlen oder einem heißen Draht mikrostrukturiert wird.

## Claims

1. A vascular stent, comprising a substrate on the basis of nitinol and, arranged thereon, at least partially a polymer having the following formula (I), 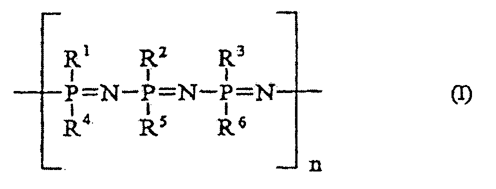 wherein n is 2 to ∞, residues R¹ to R⁶ are equal or different and represent an alkoxy, alkylsulfonyl, dialkylamino, or aryloxy radical or a heterocycloalkyl or heteroaryl radical having nitrogen as the hetero atom.

2. The vascular stent according to claim 1, wherein an ultra-thin TiO₂ layer is arranged on the surface of the substrate based on nitinol.

3. The vascular stent according to claim 1 or 2, wherein at least one of residues R¹ to R⁶ is an alkoxy radical which is substituted with at least one fluorine atom.

4. The vascular stent according to any of claims 1 to 3, wherein the polymer is poly[bis(trifluoroethoxy)phosphazene].

5. The vascular stent according to any of claims 1 to 4, wherein a layer containing an adhesion promoter is arranged between the surface of the substrate based on nitinol and the cover.

6. The vascular stent according to claim 5, wherein the adhesion promoter is a compound containing a polar terminal group, in particular an organosilicon compound.

7. The vascular stent according to claim 6, wherein the organosilicon compound is aminopropyltrimethoxysilane.

8. The vascular stent according to any of claims 1 to 7, wherein the substrate based on nitinol is fully coated with a cover based on at least one polymer having general formula (I).

9. The vascular stent according to any of the preceding claims, wherein the substrate based on nitinol is available in the form of an at least partially perforated tube.

10. The vascular stent according to any of the preceding claims, wherein the cover based on at least one polymer having general formula (I) is microstructured.

11. A process for the production of a vascular stent according to any of claims 1 to 10, comprising the steps of:
(a) providing a substrate based on nitinol,
(b) exposing the substrate based on nitinol to a plasma treatment,
(c) optionally hydroxylating the surface treated in step (b) and applying an adhesion promoter, and
(d) coating the substrate with at least one polymer according to formula (I) as defined in any of the preceding claims.

12. The process according to claim 11, wherein the plasma in step (b) is an air or oxygen plasma.

13. The process according to claim 11 or 12, wherein after step (d) the polymer coating is microstructured by means of laser, electron or X-rays or a hot wire.

## Revendications

1. Endoprothèse vasculaire comprenant un substrat à base de nitinol et un revêtement disposé dessus au moins partiellement, à base d'au moins un polymère de la formule générale suivante (I), 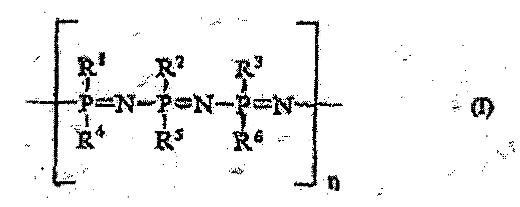 dans laquelle n représente 2 jusqu'à ∞, les radicaux R¹ à R⁶ étant identiques ou différents et représentant un radical alcoxy, un radical alkylsulfonyle, un radical dialkylamino ou un radical aryloxy ou un radical hétérocycloalkyle ou un radical hétéroaryle avec de l'azote comme hétéroatome.

2. Endoprothèse vasculaire selon la revendication 1, une couche ultra mince de TiO₂ étant disposée sur la surface du substrat à base de nitinol.

3. Endoprothèse vasculaire selon la revendication 1 ou 2, au moins un des radicaux R¹ à R⁶ étant un radical alcoxy qui est substitué avec au moins un atome de fluor.

4. Endoprothèse vasculaire selon l'une des revendications 1 à 3, le polymère étant du poly[bis(trifluoréthoxy) phosphazène].

5. Endoprothèse vasculaire selon l'une des revendications 1 à 4, une couche contenant un promoteur d'adhésion étant disposée entre la surface du substrat à base de nitinol et le revêtement.

6. Endoprothèse vasculaire selon la revendication 5, le promoteur d'adhésion étant un composé polaire contenant un groupe terminal, en particulier un composé organo-silicié.

7. Endoprothèse vasculaire selon la revendication 6, le composé organo-silicié étant de l'aminopropyltriméthoxysilane.

8. Endoprothèse vasculaire selon l'une des revendications 1 à 7, le substrat à base de nitinol étant entièrement recouvert d'un revêtement à base d'au moins un polymère de la formule générale (I).

9. Endoprothèse vasculaire selon l'une des revendications précédentes, le substrat à base de nitinol se présentant sous la forme d'un tube au moins partiellement perforé.

10. Endoprothèse vasculaire selon l'une des revendications précédentes, le revêtement à base d'au moins un polymère de la formule générale (I) étant microstructuré.

11. Procédé pour fabriquer une endoprothèse vasculaire selon l'une des revendications précédentes 1 à 10 comprenant les étapes consistant à :
(a) préparer un substrat à base de nitinol,
(b) soumettre le substrat à base de nitinol à un traitement plasma,
(c) hydroxyler le cas échéant la surface traitée dans l'étape (b) et appliquer un promoteur d'adhésion et
(d) recouvrir le substrat d'au moins un polymère selon la formule (I), comme défini dans l'une des revendications précédentes.

12. Procédé selon la revendication 11, le plasma décrit dans l'étape (b) étant un plasma d'air ou un plasma d'oxygène.

13. Procédé selon la revendication 11 ou 12, le revêtement polymère étant microstructuré selon l'étape (d) au moyen de rayons laser, de rayons électroniques ou de rayons X ou d'un fil métallique chaud.
